(19) Europäisches Patentamt
European Patent Office
Office européen des brevets

(11) Veröffentlichungsnummer: **0 103 259**
**B1**

(12) # EUROPÄISCHE PATENTSCHRIFT

(45) Veröffentlichungstag der Patentschrift: **26.02.86**

(51) Int. Cl.[4]: **C 07 D 405/14**, C 07 D 401/06

(21) Anmeldenummer: **83108778.8**

(22) Anmeldetag: **06.09.83**

(54) **4-Chinolinderivate und deren Herstellung.**

(30) Priorität: **10.09.82 CH 5393/82**

(43) Veröffentlichungstag der Anmeldung: **21.03.84 Patentblatt 84/12**

(45) Bekanntmachung des Hinweises auf die Patenterteilung: **26.02.86 Patentblatt 86/9**

(84) Benannte Vertragsstaaten: **AT BE CH DE FR GB IT LI NL**

(56) Entgegenhaltungen:
**DE - A - 2 806 909**
**US - A - 2 498 497**

**Chemical Abstracts, Vol. 96, No. 13, 29. März 1982,** ***Columbus, Ohio, USA Konno "Studies on quinolineand*** **isoquinoline derivatives" Seite 690, Spalten 1,2, Abstract-No. 104054u**

(73) Patentinhaber: **F. HOFFMANN-LA ROCHE & CO. Aktiengesellschaft, CH-4002 Basel (CH)**

(72) Erfinder: **Adam-Molina, Solange, Dr., 60 D, rue de Village-Neuf, F-68300 Rosenau (FR)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

**EP 0 103 259 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue 4-Chinolinderivate und deren Herstellung; sie betrifft insbesondere N-[6- 2,8-Bis-(trifluor-methyl)-4-chinolyl -5-hexenyl]-phthalimid und N-[4-{3-<2,8-Bis-(trifluormethyl)-4-chinolyl>-2-oxiranyl}-butyl]-phthalimid und deren Herstellung sowie die Ueberführung des letzteren in Mefloquin und dessen physiologisch verträgliche Säureadditionssalze.

Mefloquin, d,l-erythro-α-(2-Piperidyl)-2,8-bis-(trifluor-methyl)-4-chinolinmethanol, ist eine bekannte Verbindung und ein wertvoller Wirkstoff zur Bekämpfung der Malaria [siehe z.B. Antimicrobial Agents Chemother. 9, 384 (1976)]. Nach den bisher bekannten Verfahren wurde Mefloquin entweder über metallorganische Zwischenstufen [vgl. J. Med. Chem. 14, 926 (1971); deutsche Offenlegungsschriften 28 06 909 und 29 40 443] oder neuerlich unter Vermeidung derartiger Zwischenprodukte nach Verfahren hergestellt, deren letzte Stufe in einer katalytischen Hydrierung von 2-Pyridyl-2,8-bis-(trifluormethyl)-4-chino-lylketon oder 2-Pyridyl-2,8-bis-(trifluormethyl)-4-chinolin-acetoxymethan besteht [vgl. europäische Patentanmeldung 49 776].

Bei diesen Verfahren fällt neben der erythro-Form auch stets eine geringe Menge (ca. 5 - 15%) der unerwünschten threo-Form des 2-Piperidyl-4-chinolinmethanols an. Die Trennung dieses Gemisches und die Reindarstellung der erythro-Form war bisher nur durch relativ aufwendige Verfahren, die mehrmalige Umkristallisation aus Aceton/Alkohol-Gemischen, Waschen mit Aceton und Kristallisation aus Acetonitril umfasste, möglich.

Der vorliegenden Erfindung lag daher die Aufgabe zugrunde, eine möglichst niedrigstufige Synthese für Mefloquin mit hoher Gesamtausbeute zu entwickeln, bei der einerseits die Verwendung metallorganischer Zwischenstufen vermieden wird und andererseits in der letzten Stufe das Mefloquin mit hoher Stereospezifität gebildet wird und möglichst rein anfällt, so dass keine Notwendigkeit einer Abtrennung der t[illegible] von der erythro-Form des α-(2-Piperidyl)-2,8-bis-(trifluormethyl)-4-chinolinmethanols besteht.

Diese Aufgabe wurde erfindungsgemäss durch die Realisierung der im folgenden Schema dargestellten Reaktionssequenz gelöst, die über die beiden neuen Zwischenverbindungen N-[6-<2,8-Bis-(trifluormethyl)-4-chinolyl>-5-hexenyl]-phthalimid und N-[4-{3-<2,8-Bis-(trifluormethyl)-4-chinolyl>-2-oxiranyl}-buty1]-phthalimid verläuft. In diesem Reaktionsschema steht M für Natrium oder Kalium und X für Chlor, Brom oder Jod.

Das 6-Phthalimido-1-hexen, Ausgangsmaterial der erfindungsgemässen Reaktionssequenz, wird dadurch erhalten, dass man ein 6-Halogen-1-hexen (Halogen = Chlor, Brom oder Jod), vorzugsweise 6-Brom-1-hexen, in an sich bekannter Weise mit Phthalimid-natrium oder -kalium umsetzt. Die Umsetzung wird zweckmässigerweise in Gegenwart einer Base, vorzugsweise in einem basischen Lösungsmittel, z.B. N,N-Dimethylacetamid, durchgeführt.

Das 6-Phthalimido-1-hexen wird dann mit einem 4-Halogen-2,8-bis-(trifluormethyl)-chinolin (Halogen = Chlor, Brom oder

**Reaktionsschema:**

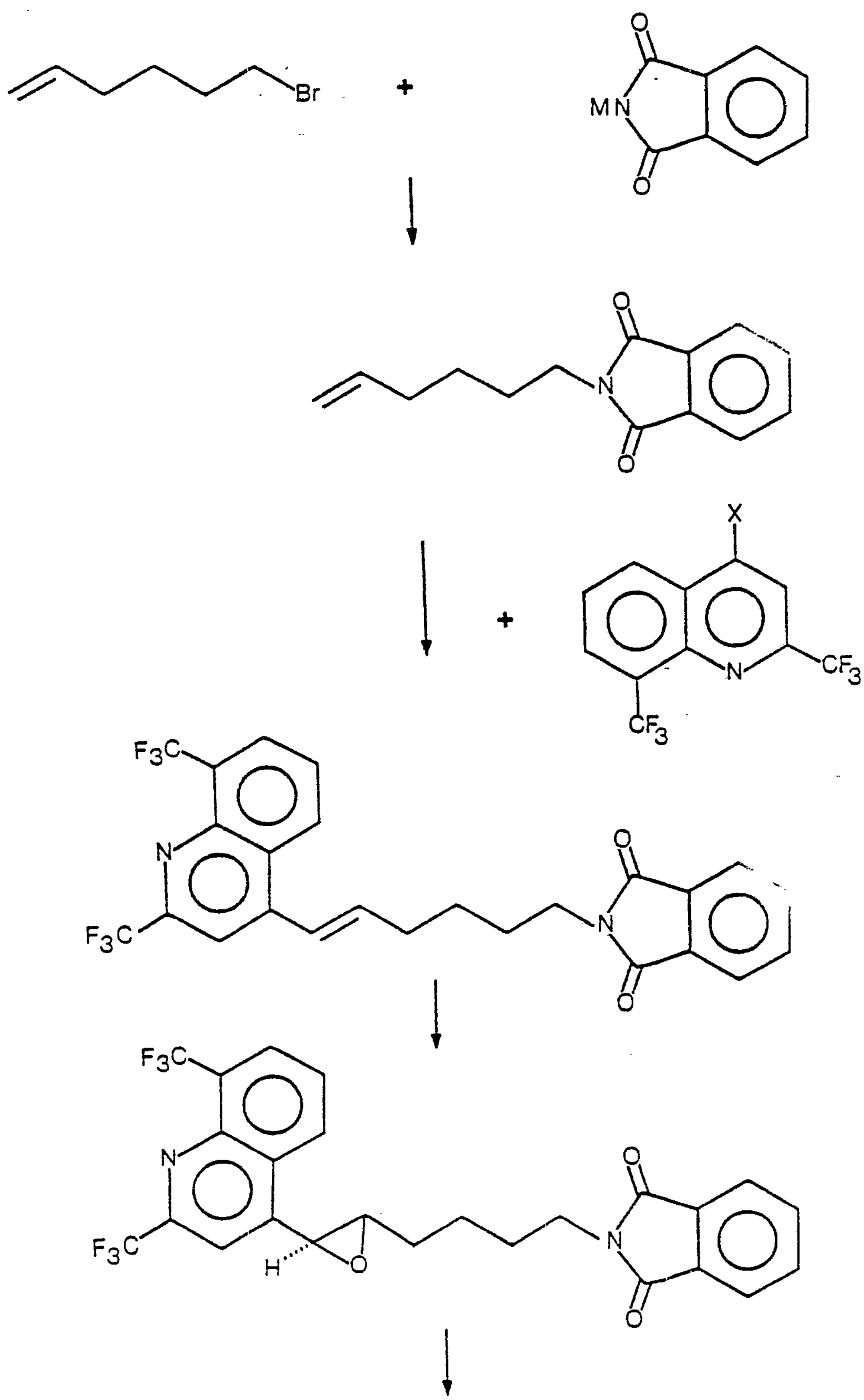

Mefloquin

Jod) zu N-[6-<2,8-Bis-(trifluormethyl)-4-chinolyl>-5-hexenyl]-phthalimid umgesetzt. Diese Reaktion findet zweckmässigerweise in einem inerten organischen Lösungsmittel wie Benzol, Toluol, Acetonitril, Hexamethylphosphortriamid, Dimethylsulfoxid, 1,3-Dimethyl-3,4,5,6-tetrahydro-2(1H)-pyrimidinon oder 1,3-Dimethyl-2-imidazolidinon, in Gegenwart einer Base, z.B. einem tert.-Alkylamin, wie Triethyl- oder Tributylamin, sowie in Gegenwart eines Palladiumsalzes, z.B. Palladiumdichlorid oder:diacetat, und eines Phosphins, z.B. Triphenylphosphin oder Tri-o-tolylphosphin, statt. Die Tatsache, dass bei dieser Umsetzung ausschliesslich das trans-Produkt entsteht, ist die Ursache für die hohe Mefloquinausbeute der erfindungsgemässen Reaktionssequenz.

Das erhaltene N-[6-<2,8-Bis-(trifluormethyl)-4-chino-lyl>-5-hexenyl]-phthalimid wird dann in einem inerten Lösungsmittel zu N [illegible] <2,8-Bis-(trifluormethyl)-4-chinolyl>-2-oxiranyl}-buty1]-phthalimid epoxydiert. Die Epoxidation kann [illegible] bekannter Weise mit den üblichen Oxidationsmitteln wie Wasserstoffperoxid (in essigsaurer oder [illegible] Lösung), Peressigsäure, Perbenzoesäure, m-Chlorperbenzoesäure oder tert.-Butylhydroperoxid [illegible] wart eines basischen Katalysators (z.B. Triton B), in einem inerten Lösungsmittel, wie Dichlormethan, 1,2-Dichlorethan, Chloroform oder Tetrahydrofuran, durchgeführt werden.

Die erfindungsgemässe Ueberführung des N-[4-{3-<2,8-Bis-(trifluormethyl)-4-chinolyl>-2-oxiranyl}-butyl]-phthalimid in Mefloquin erfolgt durch Behandlung mit Hydrazinhydrat in einem inerten Lösungsmittel, vorzugsweise einem niederen Alkanol, insbesondere Methanol oder Ethanol, bei Raum- bis Rückflusstemperatur, vorzugsweise unter Erhitzen zum Rückfluss. Arbeitet man in Gegenwart einer physiologisch verträglichen Säure, so erhält man das Mefloquin in Form des gewünschten Säureadditionssalzes. Als besonders bevorzugtes Säureadditionssalz ist das Hydrochlorid anzusehen. Die Aufarbeitung erfolgt zweckmässigerweise durch Abtrennung des Phthalylhydrazids aus dem abgekühlten Reaktionsgemisch, Einengen des Filtrats und Behandlung des Rückstands mit einem Alkohol, z.B. Methanol, Ethanol oder Isopropanol, vorzugsweise Methanol, ggf. in Gegenwart von 50-80 Vol.-% Wasser. Die Behandlung kann bei Zimmertemperatur oder - zur Erhöhung der Ausbeute - unter Kühlung auf eine Temperatur etwas über 0° C, zweckmässigerweise während 6 - 12 Stunden, erfolgen. Das Mefloquin-hydrochlorid z.B. wird auf diese Weise in reiner Form und mit nahezu quantitativer Ausbeute erhalten.

**Beispiel**

Eine Suspension von 39 g Kaliumphthalimid in 150 ml N,N-Dimethylacetamid wurde bei 20° C unter Rühren mit 27 ml 6-Brom-1-hexen versetzt. Nach 4 Stunden wurde das Reaktionsgemisch auf 300 ml Eiswasser gegossen, mit Ether extrahiert, die organische Phase mit 10%iger NaCl-Lösung gewaschen, getrocknet, filtriert und zur Trockne eingeengt. Es wurden 45 g (100%) 6-Phthalimido-1-hexen, Smp. 17-20° C, erhalten.

Ein Gemisch aus 64 g 4-Brom-2,8-bis-(trifluormethyl)-chinolin, 49 g 6-Phthalimido-1-hexen, 59 ml Tributylamin, [illegible] Tri-o-tolylphosphin und 1,7 g Palladiumdiacetat in 300 ml Hexamethylphosphortriamid wurde unter Argon [illegible] Rühren 8 Stunden auf 100° C erhitzt. Es wurde auf Eiswasser gegossen und mit Essigester extrahiert. Die [illegible]ganische Phase [illegible] mit Wasser neutral gewaschen, getrocknet und zur Trockne eingeengt. Der kristalline Rückstand wur[illegible] gewaschen. Es wurden 54,9 g (60%) N-[6-<2,8-Bis-(trifluormethyl)-4-chinolyl>-5-hexenyl]-phtha[illegible] 103-105° C, in Form weisser Kristalle erhalten.

Eine Lösung [illegible] g N-[6-<2,8-Bis-(trifluormethyl)-4-chinolyl>-5-hexenyl]-phthalimid in 300 ml Chloroform wurde unter R[illegible] mit 16,5 g 90%iger m-Chlorperbenzoesäure versetzt. Das Reaktionsgemisch wurde unter Rückfluss erhitzt. Nach Beendigung der Umsetzung wurde überschüssige Persäure mit 10%iger Natriumsulfit-Lösung zersetzt und die organische Phase mit 5%iger Natriumbicarbonat-Lösung extrahiert, mit Wasser gewaschen, getrocknet, filtriert und zur Trockne eingeengt. Nach Umkristallisation des Rückstandes aus Dichlormethan/Isopropylether (2:8, v/v) erhielt man 35,5 g (97%) N-[4-{3-<2,8-Bis-(trifluormethyl)-4-chinolyl>-2-oxiranyl}-butyl]-phthalimid, Smp. 126-128° C, in Form weisser Kristalle.

Eine Suspension von 25,4 g N-[4-{3-<2,8-Bis-(trifluor-methyl)-4-chinolyl>-2-oxiranyl}-butyl]-phthalimid in 150 ml Methanol wurde unter Rühren mit 2,65 ml Hydrazinhydrat versetzt. Das Gemisch wurde 8 Stunden unter Rückfluss erhitzt und dann unter vermindertem Druck eingeengt. Der kristalline Rückstand wurde in Ethanol suspendiert und die Suspension nach Zusatz von 11 ml einer 5 N HCl ethanolischen Lösung 1 Stunde unter Rückfluss erhitzt. Nach dem Abkühlen wurde das Phthalylhydrazid abgenutscht, das Filtrat eingeengt und der kristalline Rückstand aus Ethanol umkristallisiert. Es wurden 19 g (96%) Mefloquin-hydrochlorid, Smp. 254-256° C, in Form weisser Kristalle erhalten.

**Patentansprüche** für die Vertragsstaaten BE, CH, DE, FR, GB, IT, LI, NL,

1. N-[4-{3-<2,8-Bis-(trifluormethyl)-4-chinolyl>-2-oxiranyl}-butyl]-phthalimid.
2. N-[6-<2,8-Bis-(trifluormethyl)-4-chinolyl>-5-hexenyl]-phthalimid.
3. Verfahren zur Herstellung von N-[4-{3-<2,8-Bis-(tri-fluormethyl)-4-chinolyl>-2-oxiranyl}-buty1]-phthalimid, dadurch gekennzeichnet, dass man N-[6-<2,8-Bis-(trifluormethyl)-4-chinolyl>-5-hexenyl]-phthalimid in einem inerten Lösungsmittel epoxydiert.

4. Verfahren gemäss Anspruch 3, dadurch gekennzeichnet, dass man mit m-Chlorperbenzoesäure epoxydiert.

5. Verfahren gemäss Anspruch 3 oder 4, dadurch gekennzeichnet, dass man das N-[6-<2,8-Bis-(trifluormethyl)-4-chinolyl>-5-hexenyl]-phthalimid durch Umsetzung von 6-Phthal-imido-1-hexen mit einem 4-Halogen-2,8-bis-(trifluormethyl): chinolin (wobei Halogen Chlor, Brom oder Jod bedeutet), in einem inerten Lösungsmittel, in Gegenwart einer Base herstellt.

6. Verfahren gemäss Anspruch 5, dadurch gekennzeichnet, dass man die Umsetzung in Hexamethylphosphortriamid, in Gegenwart von o-Tolyltriphenylphosphin und Palladiumdiacetat durchführt.

7. Verfahren zur Herstellung von Mefloquin und seinen physiologisch verträglichen Säureadditionssalzen, dadurch gekennzeichnet, dass man N-[4-{3-<2,8-Bis-(trifluormethyl)-4-chinolyl>-2-oxiranyl}-butyl]-phthalimid mit Hydrazinhydrat umsetzt und das Reaktionsprodukt gewünschtenfalls in ein physiologisch verträgliches Säureadditionssalz überführt.

**Patentansprüche** für der Vertragsstaat A

1. Verfahren zur Herstellung von N-[4-{3-<2,8-Bis-(tri-fluormethyl)-4-chinolyl>-2-oXiranyl}-buty1]-phthalimid, dadurch gekennzeichnet, dass man N-[6-<2,8-Bis-(trifluormethyl)-4-chinolyl>-5-hexenyl]-phthalimid in einem inerten Lösungsmittel epoxydiert.

2. Verfahren gemäss Anspruch 1, dadurch gekennzeichnet, dass man mit m-Chlorperbenzoesäure epoxydiert.

3. Verfahren gemäss Anspruch 1 oder 2, dadurch gekennzeichnet, dass man das N-[6-<2,8-Bis-(trifluormethyl)-4-chinolyl>-5-hexenyl]-phthalimid durch Umsetzung von 6-Phthal-imido-1-hexen mit einem 4-Halogen-2,8-bis-(trifluormethyl)chinolin, (wobei Halogen Chlor, Brom oder Jod bedeutet), in einem inerten Lösungsmittel, in Gegenwart einer Base herstellt.

4. Verfahren gemäss Anspruch 3, dadurch gekennzeichnet, dass man die Umsetzung in Hexamethylphosphortriamid, in Gegenwart von o-Tolyltriphenylphosphin und Palladiumdiacetat durchführt.

5. Verfahren zur Herstellung von Mefloquin und seinen physiologisch verträglichen Säureadditionssalzen, dadurch gekennzeichnet, dass man N-[4-{3-<2,8-Bis-(trifluormethyl)-4-chinolyl>-2-oxiranyl}-butyl]-phthalimid mit Hydrazinhydrat umsetzt und das Reaktionsprodukt gewünschtenfalls in ein physiologisch verträgliches Säureadditionssalz überführt.

**Claims** for the Contracting States BE, CH, DE, FR, GB, IT, LI, NL

1. N-[4-{3-<2,8-Bis-(trifluoromethyl)-4-quinolyl>-2-oxiranyl}-butyl)-phthalimide.

2. N-[6-<2,8-Bis-(trifluoromethyl)-4-quinolyl>-5-hexenyl]-phthalimide.

3. A process for the manufacture of N-[4-{3-<2,8-bis-trifluoromethyl)-4-quinolyl>-2-oxiranyl}-butyl]-phthalimide, characterized by epoxidizing N-[6-<2,8-bis-(trifluoromethyl)-4-quinolyl>-5-hexenyl]-phthalimide in an inert solvent.

4. A process in accordance with claim 3, characterized in that the epoxidation is carried out with m-chloroperbenzoic acid.

5. A process in accordance with claim 3 or 4, characterized in that the N-[6-<2,8-bis-(trifluoromethyl)-4-quinolyl>-5-hexenyl]-phthalimide is prepared by reacting 6-phthalimido-1-hexene with a 4-halogen-2,8-bis-(trifluoromethyl)-quinoline (in which halogen signifies chlorine, bromine or iodine) in an inert solvent in the presence of a base.

6. A process in accordance with claim 5, characterized in that the reaction is carried out in hexamethylphosphortriamide in the presence of o-tolyltriphenylphosphine and palladium diacetate.

7. A process for the manufacture of mefloquin and its physiologically compatible acid addition salts, characterized by reacting N-[4-{3-<2,8-bis-(trifluoro-methyl)-4-quinolyl>-2-oxiranyl}-butyl]-phthalimide with hydrazine hydrate and, if desired, converting the reaction product into a physiologically compatible acid addition salt.

**Claims** for the Contracting State AT

1. A process for the manufacture of N-[4-{3-<2,8-bis-trifluoromethyl)-4-quinolyl>-2-oxiranyl}-butyl]-phthalimide, characterized by epoxidizing N-[6-<2,8-bis-(trifluoromethyl)-4-quinolyl>-5-hexenyl]-phthalimide in an inert solvent.

2. A process in accordance with claim 1, characterized in that the epoxidation is carried out with m-chloroperbenzoic acid.

3. A process in accordance with claim 1 or 2, characterized in that the N-[6-<2,8-bis-(trifluoromethyl)-4-quinolyl>-5-hexenyl]-phthalimide is prepared by reacting 6-phthalimido-1-hexene with a 4-halogen-2,8-bis-(trifluoromethyl)-quinoline (in which halogen signifies chlorine, bromine or iodine) in an inert solvent in the

presence of a base.

4. A process in accordance with claim 3, characterized in that the reaction is carried out in hexamethylphosphortriamide in the presence of o-tolyltriphenylphosphine and palladium diacetate.

5. A process for the manufacture of mefloquin and its physiologically compatible acid addition salts, characterized by reacting N-[4-{3-<2,8-bis-(trifluoro-methyl)-4-quinolyl>-2-oxiranyl}-butyl]-phthalimide with hydrazine hydrate and, if desired, converting the reaction product into a physiologically compatible acid addition salt.

**Revendications** pour les Etats Contractants: BE, CH, DE, FR, GB, IT, LI, NL

1 - Le N-[4-{3-<2,8-bis-(trifluorométhyl)-4-quinoléyl>-2-oxirannyl}-butyl]-phtalimide.

2 - Le N-[6-<2,8-bis-(trifluorométhyl)-4-quinoléyl>-5-hexényl] -phtalimide.

3 - Procédé de préparation du N-[4-{ 3- <2,8-bis-(trifluorométhyl)-4-quinoléyl> -2-oxirannyl}-butyl>-phtalimide, caractérisé en ce que l'on époxyde le N-[6-< 2,8-bis-(trifluorométhyl)-4-quinoléyl>-5-hexényl] -phtalimide dans un solvant inerte.

4 - Procédé selon la revendication 3, caractérisé en ce que l'on époxyde à l'aide de l'acide m-chloroperbenzoïque.

5 - Procédé selon la revendication 3 ou 4, caractérisé en ce que l'on prépare le N-[-6-< 2,8-bis-(trifluorométhyl)-4-quinoléyl> -5-hexényl]-phtalimide par réaction du 6-phtalimido-1-hexène avec une 4-halogéno-2,8-bis-(trifluorométhyl)-quinoléine (dans laquelle l'halogène est le chlore, le brome ou l'iode) dans un solvant inerte, en présence d'une base.

6 - Procédé selon la revendication 5, caracterisé en ce que l'on effectue la réaction dans l'hexaméthylphosphorotriamide en présence d'o-tolyltriphénylphosphine et de diacétate de palladium.

7 - Procédé de préparation du Mefloquin et de ses sels formés par addition avec des acides acceptables pour l'usage pharmaceutique, caractérisé en ce que l'on fait réagir le N-[4- {3-<2,8-bis-(trifluorométhyl)-4-quino-léyl] -2-oxirannyl} -butyl]-phtalimide avec l'hydrate d'hydrazine, après quoi, si on le désire, on convertit le produit de réaction en un sel par addition avec un acide acceptable pour l'usage pharmaceutique.

**REVENDICATIONS** pour l'Etat Contractant: AT

1 - Procédé de préparation du N-[4-{3-<2,8-bis-(trifluorométhyl)-4-quinoléyl> -2-oxirannyl}-butyl]-phtalimide, caractérisé en ce que l'on époxyde le N-[6-<2,8-bis-(trifluorométhyl)-4-quinoléyl>-5-hexényl]-phtalimide dans un solvant inerte.

2 - Procédé selon la revendication 1, caractérisé en ce que l'on époxyde à l'aide de l'acide m-chloroperbenzoïque.

3 - Procédé selon la revendication 1 ou 2, caractérisé en ce que l'on prépare le N-[6- <2,8-bis-(trifluorométhyl)-4-quinoléyl> -5-hexényl]phtalimide par réaction du 6-phtalimido-1-hexène avec une 4-halogéno-2,8-bis-(trifluorométhyl)-quinoléine (dans laquelle l'halogène est le chlore, le brome ou l'iode) dans un solvant inerte, en présence d'une base.

4 - Procédé selon la revendication 3, caractérisé en ce que l'on effectue la réaction dans l'hexaméthylphosphorotriamide, en présence d'o-tolyltriphénylphosphine et de diacétate de palladium.

5 - Procédé de préparation du Mefloquin et de ses sels formés par addition avec des acides acceptables pour l'usage pharmaceutique, caractérisé en ce que l'on fait réagir le N-[4-{3-<2,8-bis-(trifluorométhyl)-4-quinoléyl>-2-oxirannyl}-butyl]-phtalimide avec l'hydrate d'hydrazine, après quoi, si on le désire, on convertit le produit de réaction en un sel par addition avec un acide acceptable pour l'usage pharmaceutique.